(19) Europäisches Patentamt
European Patent Office
Office européen des brevets

(11) **EP 3 429 544 B1**

(12) **EUROPÄISCHE PATENTSCHRIFT**

(45) Veröffentlichungstag und Bekanntmachung des Hinweises auf die Patenterteilung:
**19.04.2023   Patentblatt 2023/16**

(21) Anmeldenummer: **17710303.3**

(22) Anmeldetag: **15.03.2017**

(51) Internationale Patentklassifikation (IPC):
***A61J 3/00*** *(2006.01)*

(52) Gemeinsame Patentklassifikation (CPC):
**A61J 3/002;** A61J 2200/74

(86) Internationale Anmeldenummer:
**PCT/EP2017/056106**

(87) Internationale Veröffentlichungsnummer:
**WO 2017/158011 (21.09.2017 Gazette 2017/38)**

(54) **VERFAHREN ZUR HERSTELLUNG EINER MEDIZINISCHEN ZUBEREITUNG UNTER VERWENDUNG EINER SCHLAUCHPUMPE**

METHOD FOR PRODUCING A MEDICAL PREPARATION USING A HOSE PUMP

PROCEDE DE FABRICATION D'UNE PREPARATION MEDICAMENTEUSE A L'AIDE D'UNE POMPE TUBULAIRE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **15.03.2016   EP 16160328**

(43) Veröffentlichungstag der Anmeldung:
**23.01.2019   Patentblatt 2019/04**

(73) Patentinhaber: **Fresenius Kabi Deutschland GmbH**
**61352 Bad Homburg (DE)**

(72) Erfinder:
• BIEHL, Martin
  66606 St. Wendel (DE)
• HOCK, Michael
  35516 Münzenberg (DE)
• SCHAAKE, Henrik
  61348 Bad Homburg (DE)
• BOHM, Martin
  39110 Magdeburg (DE)
• SCHÖBEL, Ulla
  06366 Köthen (DE)

(74) Vertreter: **Fresenius Kabi Deutschland GmbH**
**Patent Department**
**Borkenberg 14**
**61440 Oberursel (DE)**

(56) Entgegenhaltungen:
| | |
|---|---|
| WO-A1-87/07236 | US-A- 4 473 173 |
| US-A- 5 046 569 | US-A- 5 228 485 |
| US-A- 5 697 407 | US-A1- 2006 245 964 |
| US-A1- 2013 189 120 | |

EP 3 429 544 B1

**Beschreibung**

Gebiet der Erfindung

Die Erfindung betrifft ein Verfahren und eine Anlage zur Herstellung einer medizinischen Zubereitung, wobei eine Schlauchpumpe zum Transferieren von Flüssigkeiten aus Quellbehältern in einen Zielbehälter verwendet wird. insbesondere betrifft die Erfindung ein Verfahren, mittels dessen Infusionsbeutel und/oder Spritzen zur parenteralen Ernährung befüllt werden, sowie eine zugehörige Anlage.

Hintergrund der Erfindung

Zubereitungen zur parenteralen Ernährung werden z.B. in Apotheken oder Kliniken patientenspezifisch hergestellt. Es handelt sich dabei um Gemische aus verschiedenen Grundnahrungsstoffen, Spurenelementen und Vitaminen, ggf. auch zusammen mit einem Arzneimittel, die individuell in einen Infusionsbeutel transferiert werden.

Hierfür werden sogenannte TPN-Compounder (TPN = Total Parenteral Nutrition) verwendet. Aus der Praxis bekannte und im Markt befindliche Anlagen, wie beispielsweise das System MultiComp® der Firma Fresenius, umfassen eine computergesteuerte Pumpeneinheit, mittels der die Bestandteile der Zusammensetzung aus verschiedenen Quellbehältern in einen auf einer Waage befindlichen Zielbehälter überführt werden.

Die Anforderungen an die Sicherheit beim Herstellen derartiger medizinischer Zubereitungen sind hoch. insbesondere soll eine hohe Genauigkeit der Dosierung aller Bestandteile sichergestellt sein.

Zur Überprüfung der Dosierung kann der Zielbehälter gewogen werden. Ein Compounding-System, in dem der Zielbehälter gewogen wird, ist im Dokument US 5,697,407 beschrieben.

Problematisch ist, dass die herzustellenden medizinischen Zubereitungen Komponenten mit Hauptbestandteilen wie Wasser, Fett, Zucker und Aminosäuren umfassen, welche in recht großer Menge zugeführt werden. Daneben gibt es Komponenten, die beispielsweise bestimmte Vitamine, Mineralstoffe oder auch ein Arzneimittel umfassen, welche in wesentlicher geringerer Menge, insbesondere im ml-Bereich, zugeführt werden müssen. Derartige Bestandteile werden auch als Mikromengen bezeichnet.

Aufgabe der Erfindung

Der Erfindung liegt demgegenüber die Aufgabe zugrunde, ein Verfahren zur Herstellung einer medizinischen Zubereitung bereit zu stellen, bei welchem, vorzugsweise mittels einer Schlauchpumpe, eine genaue Dosierung der einzelnen Bestandteile einer medizinischen Zubereitung ermöglicht wird.

Zusammenfassung der Erfindung

Die Aufgabe der Erfindung wird durch ein Verfahren zur Herstellung einer medizinischen Zubereitung nach Anspruch 1 gelöst.

Bevorzugte Ausführungsformen und Weiterbildungen der Erfindung sind dem Gegenstand der abhängigen Ansprüche, der Beschreibung sowie den Zeichnungen zu entnehmen.

Die Erfindung betrifft zunächst ein Verfahren zur Herstellung einer medizinischen Zubereitung. Insbesondere betrifft die Erfindung ein Verfahren zur Herstellung einer Zubereitung zur parenteralen Ernährung.

Dabei werden einer Mehrzahl von Quellbehältern Flüssigkeiten entnommen und in einen Zielbehälter transferiert. Die Herstellung erfolgt automatisiert, wobei der Benutzer der für das Verfahren verwendeten Anlage die im Zielbehälter gewünschte Zusammensetzung eingeben kann oder diese aus einer Datenbank mit einer Mehrzahl von Rezepten auswählen kann.

Den einzelnen Quellbehältern wird in einer vorgegebenen Reihenfolge jeweils eine definierte Menge Flüssigkeit entnommen, im Folgenden auch als "Dosierschritt" bezeichnet. Nach Abschluss sämtlicher für einen Zielbehälter vorgesehener Dosierschritte ist definitionsgemäß ein "Befüllvorgang" beendet.

Es kann Bestandteile geben, welche nicht in direkten Kontakt oder nur in einer bestimmten Reihenfolge in Kontakt kommen dürfen.

Typischerweise besteht, wie eingangs ausgeführt, eine derartige medizinische Zubereitung aus Hauptbestandteilen, die in größerer Menge zugeführt werden, und sog. "Mikromengen", die insbesondere Vitamine, Mineralstoffe oder Arzneimittelkomponenten enthalten können.

Zum Transferieren wird vorzugsweise ein als Einweg-Bauteil ausgebildetes "Transfer-Set" verwendet, welches den Schlauch umfasst, der in die Schlauchpumpe eingelegt wird. Das Transfer-Set umfasst des Weiteren Anschlussschläuche für die Quellbehälter sowie einen Anschluss für den Zielbehälter. Weiter umfasst das Transfer-Set vorzugsweise eine Ventileinheit, mittels der die Anschlüsse zu den einzelnen Quellbehältern geöffnet und geschlossen werden können.

Vorzugsweise ist während jedes einzelnen Dosierschritts immer nur ein einziges Ventil, welches zu einem Quellbehälter führt, geöffnet. Es wird also immer nur einem Quellbehälter Flüssigkeit entnommen.

Neben den Hauptbestandteilen der medizinischen Zubereitung und den Mikromengen gibt es bei jeder Zubereitung eine sogenannte universelle Flüssigkeit, auch als "Universal Ingredient" (UI) bezeichnet. Diese Flüssigkeit darf ohne unerwünschte Nebenwirkung mit jeder anderen Zutat in direkten Kontakt kommen und wird in jeder Zubereitung in einer relativ großen Menge verwendet, insbesondere zum Auffüllen der Zubereitung auf die gewünschte Gesamtmenge. Vorzugsweise handelt es sich bei der universellen Flüssigkeit zumeist um isotonisches Wasser.

Die für das Verfahren verwendete Schlauchpumpe weist einen Bereich mit linearer Kennlinie und einen Bereich mit nicht linearer Kennlinie auf.

Unter einem Bereich mit linearer Kennlinie wird der Winkelbereich eines Pumpenrades verstanden, in welchem die Pumpleistung, also das Volumen gegenüber dem Drehwinkel eines Pumpenrades der Schlauchpumpe, konstant ist. Das geförderte Volumen ist zum Drehwinkel der Pumpe proportional.

Es gibt einen saugseitig linearen Bereich. Dies ist der Bereich, in welchem eine saugseitige Rolle der Schlauchpumpe in Eingriff mit dem Schlauch ist und keine Rolle neu in den Eingriff mit dem Schlauch geht. Im saugseitig linearen Bereich ist der Drehwinkel zum saugseitig geförderten Volumen proportional.

Weiter gibt es einen druckseitig linearen Bereich, in dem der Drehwinkel zum druckseitig geförderten Volumen proportional ist. Die druckseitige Rolle der Schlauchpumpe ist dabei im Eingriff mit dem Schlauch und keine Rolle geht aus dem Eingriff mit dem Schlauch heraus.

Es versteht sich, dass der druckseitige lineare Bereich der Kennlinie phasenverschoben zu dem saugseitig linearen Bereich der Kennlinie ist.

Bei einer Schlauchpumpe gehen bei bestimmten Phasenwinkeln die Rollen des Pumpenrades in den Eingriff und bei anderen Phasenwinkeln aus dem Eingriff heraus. Dabei ist zu jedem Zeitpunkt mindestens eine Rolle im Eingriff, die Pumpe ist zu keinem Zeitpunkt "offen". Eine Rollenpumpe hat daher theoretisch keinen Schlupf, also keine Abweichung zwischen Drehwinkel und geförderter Menge.

Wenn eine Rolle neu in den Eingriff geht, dann verringert sich das Volumen des in die Pumpe eingelegten Schlauchs, wenn eine Rolle aus dem Eingriff herausgeht, vergrößert sich das Volumen wieder. In der Folge ist die Pumpleistung, also das geförderte Volumen pro Drehwinkel, nicht konstant. Die Pumpe "pulsiert". Dieses Pulsieren tritt sowohl an der Saug- als auch an der Druckseite der Pumpe auf.

Diese nichtlineare Kennlinie sowohl an der Druck- als auch an der Saugseite der Schlauchpumpe ist nachteilig für die Dosiergenauigkeit, was besonders nachteilig ist, wenn mit einer einzigen Schlauchpumpe sowohl Hauptbestandteile in recht großer Menge als auch Mikromengen dosiert werden sollen.

Gemäß der Erfindung wird die Schlauchpumpe zur Dosierung aus zumindest einem Quellbehälter derart in eine Stellung gebracht, dass die gesamte Dosierung aus diesem Quellbehälter in einem Bereich mit linearer Kennlinie erfolgt.

Der Erfindung liegt die Erkenntnis zugrunde, dass die Dosierung von Mikromengen auch mit einer Schlauchpumpe mit hoher Genauigkeit möglich ist, wenn während des gesamten Dosierschritts die Schlauchpumpe ausschließlich im Bereich mit linearer Kennlinie bewegt wird.

Es versteht sich, dass hierfür die Menge der bei diesem Dosierschritt zu dosierenden Flüssigkeit derart gering sein muss, dass die gesamte zu dosierende Flüssigkeit in einem Winkelbereich des Pumpenrades der Schlauchpumpe gefördert werden kann, indem dieses den linearen Bereich nicht verlässt.

Um den Winkel, in welchem ein Pumpenrad der Schlauchpumpe steht, feststellen zu können, umfasst die Schlauchpumpe vorzugsweise einen Drehwinkelgeber.

Vorzugsweise wird die Schlauchpumpe in eine Stellung gebracht, in welcher die saugseitige Kennlinie der Schlauchpumpe linear ist. Bei der Dosierung von Mikromengen ist vor allem die dem Quellbehälter entnommene Menge an Flüssigkeit entscheidend und es wird daher der saugseitig vorhandene lineare Bereich der Schlauchpumpe verwendet, um die entnommene Menge möglichst exakt zu dosieren.

Um die Schlauchpumpen in die gewünschte Stellung, also den Bereich mit linearer Kennlinie, zu bringen, kann Flüssigkeit aus einem anderen Quellbehälter entnommen werden als aus dem, aus dem dosiert werden soll. Insbesondere kann zum Bewegen des Pumpenrades, beispielsweise an den Anfang des saugseitigen linearen Bereichs, als Quellbehälter ein Quellbehälter mit der vorstehend beschriebenen universellen Flüssigkeit (UI) verwendet werden. Während die Pumpe im nicht linearen Bereich arbeitet, wird also aus dem Quellbehälter mit universeller Flüssigkeit Medium entnommen.

Im Bereich der linearen Kennlinie wird vorzugsweise eine Kleinstmenge mit einem Volumen von unter 10 ml, bevorzugt von unter 5 ml, besonders bevorzugt von kleiner oder gleich 3 ml während eines Dosierschritts gefördert. Es versteht sich, dass die Schlauchpumpe derart dimensioniert sein muss, dass im Bereich der linearen Kennlinie das gesamte zuvor genannte Volumen gefördert werden kann.

Reicht das während eines einzigen Dosierschritts im linearen Bereich förderbaren Volumen nicht aus, ist es gemäß einer Ausführungsform der Erfindung auch vorgesehen, Flüssigkeit aus einem Quellbehälter in mehreren Dosierschritten zu entnehmen, wobei zwischen diesen einzelnen Dosierschritten die Schlauchpumpe jeweils an den Anfang eines linearen Bereichs gefahren wird.

In den Dosierschritten, in denen die Hauptbestandteile der medizinischen Zubereitung transferiert werden und bei denen die Dosiergenauigkeit eine weniger große Rolle spielt, kann die Schlauchpumpe in herkömmlicher Weise betrieben werden, also während des jeweiligen Dosierschrittes sowohl der nicht lineare als auch der lineare Bereich der saug- und/oder druckseitigen Kennlinie der Schlauchpumpe durchfahren werden.

Insbesondere wird in zumindest einem weiteren Dosierschritt eine Menge mit einem Volumen von über 15 ml, vorzugsweise über 20 ml gefördert, wobei die Schlauchpumpe sowohl im Bereich mit linearer Kennlinie als auch in einem Bereich mit nichtlinearer Kennlinie betrieben wird. So werden vorzugsweise die Hauptbestandteile der medizinischen Zubereitung dosiert.

Durch die Erfindung kann insbesondere ermöglicht werden, dass sämtliche Dosierschritte beim Herstellen der medizinischen Zubereitung mittels einer einzigen Schlauchpumpe mit hoher Genauigkeit durchgeführt werden.

Bei einer Weiterbildung der Erfindung wird anhand der dem jeweiligen Quellbehälter zu entnehmenden Menge an Flüssigkeit die hierfür erforderliche Drehung des Pumpenrades auf Basis der saugseitigen Kennlinie der Schlauchpumpe berechnet.

Über die Drehung der Schlauchpumpe, insbesondere über den Winkel und die Anzahl an Umdrehungen eines Pumpenrades der Schlauchpumpe, kann bei jedem Dosierschritt die dem jeweiligen Quellbehälter entnommene Menge an Flüssigkeit bestimmt werden. Anhand der vorgegebenen zu entnehmenden Menge an Flüssigkeit wird so die Pumpe angesteuert und der erforderliche Drehwinkel für einen Dosierschritt berechnet.

Bei der Dosierung und damit bei der Ansteuerung der Schlauchpumpe bei jedem Dosierschritt wird also nicht von einer konstanten Förderleistung ausgegangen, sondern es wird auf Basis einer zuvor bestimmten und gespeicherten Kennlinie die saugseitig vorhandene Schwankung der Pumpleistung einberechnet.

Dies verbessert die Dosiergenauigkeit auch bei Dosierschritten, bei denen die Schlauchpumpe nicht ausschließlich im linearen Bereich betrieben wird.

Vorzugsweise wird bei bzw. nach jedem Dosierschritt der Zielbehälter gewogen und so die Menge der jeweils transferierten Flüssigkeit überprüft.

Vorzugsweise wird für diese Überprüfung auf Basis des Gewichts des Zielbehälters aber nicht die berechnete Menge auf Basis der saugseitigen Kennlinie der Schlauchpumpe zugrunde gelegt, sondern es wird unter Berücksichtigung der druckseitigen Kennlinie der Schlauchpumpe berechnet, welche Menge an Flüssigkeit bei Drehung des Pumpenrades um den zuvor berechneten Drehwinkel in den Zielbehälter transferiert wurde.

Stimmt diese berechnete Menge in den Zielbehälter transferierter Flüssigkeit mit dem Ergebnis der Wägung des Zielbehälters überein, so kann der jeweilige Dosierschritt als korrekt betrachtet werden. Stimmen die Ergebnisse dagegen nicht überein bzw. liegen diese außerhalb eines vorgegebenen Toleranzbereiches, kann anlagenseitig ein Fehler, zum Beispiel auf einem Display, angezeigt werden.

Je nach Art und Erheblichkeit des Unterschiedes zwischen berechneter und gewogener Menge kann der Benutzer der Anlage, beispielsweise durch Anzeige auf einem Display, veranlasst werden, den Zielbehält zu verwerfen und einen neuen Zielbehälter zu befüllen und/oder die Anlage zu kalibrieren.

Insbesondere bei der Dosierung von Mikromengen kann es sein, dass nach Entnahme einer vorgegebenen Menge an Flüssigkeit aus einem Quellbehälter die Flüssigkeit nicht unmittelbar in dem Zielbehälter ankommt, sondern sich zunächst im Transfer-Set, beispielsweise im in die Schlauchpumpe eingelegten Schlauch, befindet. Die Flüssigkeit, die sich im Transfer-Set vor dieser Flüssigkeit befindet und die nunmehr in den Zielbehälter gedrückt wird, kann eine andere Dichte haben. Daher kann allein die Gewichtszunahme des Zielbehälters nicht als hinreichend genaues Maß der transferierten Menge verwendet werden.

Bei einer Ausführungsform der Erfindung wird die Menge der von der Schlauchpumpe geförderten Flüssigkeit berechnet. Es wird bei der Wägung des Zielbehälters die Abfolge und Menge verschiedener Flüssigkeiten im Zulauf des Zielbehälters berücksichtigt, um bei der Überprüfung beim Wiegen die Dichte der Flüssigkeiten mit einzuberechnen.

Diese Ausführungsform der Erfindung beruht auf der Erkenntnis, dass die Genauigkeit der Überprüfung bei jedem Dosierschritt erhöht wird, indem die Dichte, also das spezifische Gewicht der jeweiligen in den Zielbehälter transferierten Flüssigkeit, berücksichtigt wird.

Bei dieser Berechnung wird, theoretisch betrachtet, der Zulauf des Zielbehälters in Abschnitte unterteilt, in denen sich jeweils Flüssigkeit mit einer anderen Dichte befindet.

Vorzugsweise unter Berücksichtigung der druckseitigen Kennlinie der Schlauchpumpe kann nunmehr vorhergesagt werden, welche Flüssigkeit bzw. Flüssigkeiten bei einem Dosierschritt in den Zielbehälter eingeleitet werden.

Diesem Prinzip liegt die Betrachtung zugrunde, dass sämtliche den Quellbehältern entnommenen Flüssigkeiten letztendlich im Zielbehälter ankommen. Da das Volumen der Strecke vom Quellbehälter bzw. vom Ventil, ab dem die Flüssigkeit des jeweiligen Quellbehälters in die Ventileinheit fließt, bis zum auf der Waage befindlichen Quellbehälter bekannt ist, kann berechnet werden, welche Flüssigkeit oder welche Flüssigkeiten bei einem Dosierschritt im Zielbehälter ankommt oder ankommen.

Das Volumen wird durch die Ventileinheit ab der Position des jeweiligen Ventils des Quellbehälters sowie durch den durch die Schlauchpumpe geführten Schlauch bestimmt, der die Ventileinheit mit dem Zielbehälter verbindet.

Bei der Überprüfung des jeweiligen Dosierschritts durch Wägung des Zielbehälters wird daher nicht die Dichte der bei dem jeweiligen Dosierschritt entnommenen Flüssigkeit zugrunde gelegt, sondern die Dichte der Flüssigkeit oder der Flüssigkeiten, die in den Zielbehälter eingeleitet werden. Die Dichte der eingeleiteten Flüssigkeit kann sich aufgrund des Volumens des Zulaufes und der Schlauchpumpe zumindest am Anfang des Dosierschritts unterscheiden.

Es versteht sich, dass die in einem Zulauf und/oder in einem Schlauch der Schlauchpumpe angeordneten Flüssigkeiten nicht exakt entsprechend dieses Berechnungsmodels voneinander separiert sind, sondern sich verschiedene Flüssigkeiten im Bereich der Grenzfläche vermischen. Es hat sich aber gezeigt, dass diese Vermischungseffekte im Allgemeinen oder in einer Näherung vernachlässigt werden können.

Bei einer Ausführungsform der Erfindung wird der Zielbehälter bei jedem einzelnen Dosierschritt gewogen und so die Menge der in den Zielbehälter transferierten Flüssigkeit bei jedem einzelnen Dosierschritt überprüft.

Eine Überprüfung jedes einzelnen Dosierschritts wird vorzugsweise auch bei Mikromengen dadurch ermöglicht, dass die Menge der bei einem Dosierschritt in den Zielbehälter transferierten Flüssigkeit unter Berücksichtigung der druckseitigen Kennlinie der Schlauchpumpe berechnet wird.

Bei herkömmlichen Anlagen zur Zubereitung parenteraler Ernährung kann mit einer genau arbeitenden Wägezelle zwar am Ende des Befüllvorgangs, also nach Abschluss sämtlicher Dosierschritte, überprüft werden, ob die Gewichtszunahme des Zielbehälters mit der zu dosierenden Sollmenge der Einzelbestandteile übereinstimmt.

Zumindest bei Mikromengen ist jedoch eine hinreichend genaue Beurteilung jedes einzelnen Dosierschritts aufgrund der druckseitig nicht linearen Kennlinie grundsätzlich nicht möglich.

Durch die Berücksichtigung der druckseitigen Kennlinie und/oder durch den Betrieb der Schlauchpumpe im linearen Bereich bei der Dosierung von Mikromengen kann dagegen eine Überprüfung des einzelnen Dosierschritts durch Wiegen des Zielbehälters, insbesondere auch bei Mikromengen, vorgenommen werden.

Hierdurch wird die Sicherheit, dass die Zusammensetzung der medizinischen Zubereitung den Vorgaben entspricht, erhöht.

Bei der Dosierung von Mikromengen kann es zudem zu Okklusionen kommen, die anlagenseitig schwer zu erfassend sind. Sofern beispielsweise der von einem Quellbehälter zur Schlauchpumpe führende Schlauch verstopft ist, fördert bei einer geringen Menge, insbesondere bei einer Menge von unter 3 ml, die Schlauchpumpe dennoch Flüssigkeit in den Zielbehälter, da sich die flexiblen Schläuche des Transfer-Sets zusammenziehen können. Wird nunmehr im Anschluss das Ventil zu einem anderen Quellbehälter geöffnet, so entspannt sich der Schlauch, indem dieser Flüssigkeit aus dem anderen Quellbehälter ansaugt.

Dieser Effekt kann unter Umständen dazu führen, dass am Ende aller Dosierschritte die mit dem Wiegen des Zielbehälters überprüfte Gesamtmenge stimmt, eine einzelne Mikromenge aber in völlig falscher Dosierung oder gar nicht vorliegt.

Bei einer Weiterbildung der Erfindung wird daher mittels eines Flusssensors die Förderleistung der Schlauchpumpe überprüft. Vorzugsweise ist der Flusssensor saugseitig angeordnet. Es kann insbesondere ein Flusssensor vorgesehen sein, in welchen ein Schlauch des Transfer-Sets eingelegt wird.

Derartige Flusssensoren sind bekannt. Es hat sich herausgestellt, dass sich diese aber nicht eignen, um die Durchflussmenge auch bei sehr niedriger Fließgeschwindigkeit exakt zu bestimmen.

Im Falle einer Verstopfung oder im Falle eines sich nicht öffnenden Ventils des Transfer-Sets kann über den Flusssensor aber eine derart hohe Abweichung von einem Sollwert festgestellt werden, dass daraus geschlossen werden kann, dass die Durchflussmenge zur gegenwärtigen theoretischen Förderleistung der Pumpe nicht plausibel ist.

Das Verfahren kann sodann abgebrochen werden und der Benutzer der Anlage über eine Fehlermeldung informiert werden.

Bei einer Weiterbildung der Erfindung wird ein Blasensensor (Bubbel-Detector) verwendet, um in einem Zulauf im Zielbehälter zu überprüfen, dass keine Blasen im Schlauch gefördert werden.

Dieser Blasensensor, welcher beispielsweise als Ultraschall-Sensor ausgebildet sein kann, befindet sich vorzugsweise druckseitig zur Schlauchpumpe. Es handelt sich insbesondere um einen Sensor, in welchen der Schlauch eines Transfer-Sets eingelegt werden kann.

Im Falle des Vorhandenseins von Blasen über einem Schwellwert kann ebenfalls das Verfahren gestoppt und der Benutzer mittels einer Fehlermeldung informiert werden.

Bei einer bevorzugten Ausführungsform der Erfindung wird der Dosierfaktor der Schlauchpumpe in einem vorgeschalteten Kalibrierschritt mittels Wiegen eines Zielbehälters bestimmt.

Der Dosierfaktor ist das Volumen, welches bei Förderung einer bestimmten Flüssigkeit, insbesondere bei Förderung von Wasser, bei einer bestimmten Geschwindigkeit des Pumpenrades und bei einer vollen Pumpenumdrehung gefördert wird. Der Dosierfaktor hängt unter anderem von Toleranzen des in die Pumpe eingelegten Schlauchs ab. Dieser Dosierfaktor kann bei Inbetriebnahme der Anlage beim Befüllen eines Zielbehälters kalibriert werden, um die Ansteuerung der Schlauchpumpe einem neu verwendeten Transfer-Set anzupassen.

Es ist insbesondere vorgesehen, dass bei Inbetriebnahme der Anlage zur Herstellung der medizinischen Zubereitung ein erster Zielbehälter verwendet wird, welcher im Anschluss verworfen wird, ein sogenannter "waste bag". Dieser waste bag ("Abfallbehälter") wird mittels des Transfer-Sets angeschlossen und es werden die zu sämtlichen Quellbehältern führenden Schläuche entlüftet, indem jeweils eine hierfür erforderliche Menge an Flüssigkeit entnommen wird.

Zur Bestimmung des Dosierfaktors kann Flüssigkeit, vorzugsweise Wasser, in den waste bag gefördert und dabei der Dosierfaktor bestimmt werden. Nach Verwerfen des waste bag wird nunmehr dieser Dosierfaktor für die Berechnung der von der Pumpe bei weiteren Dosierschritten geförderten Menge zugrunde gelegt.

Es versteht sich, dass der Dosierfaktor wiederum mit der vorstehend beschriebenen Berücksichtigung des nicht linearen Bereichs der saugseitigen und druckseitigen Kennlinie der Schlauchpumpe ins Verhältnis zu setzen ist.

Weiter hängt die Pumpleistung einer Schlauchpumpe unter anderem auch vom zu fördernden Medium, insbesondere von der Viskosität der zu fördernden Flüssigkeit, ab. Diese Abhängigkeit kann, wie es bei einer Ausführungsform der Erfindung vorgesehen ist, ebenfalls bei der Berechnung der geförderten Mengen berücksichtigt werden.

Bei Wasser kann ein Flussfaktor von 1,0 angesetzt werden. Bei anderen Medien, wie beispielsweise Glukose, nimmt dieser Flussfaktor höhere Werte, beispielsweise Werte bis 1,1 an. Dies kann bei der Berechnung der geförderten Mengen, insbesondere der geförderten Mengen an Hauptbestandteilen, berücksichtigt werden, indem der Flussfaktor in die Berechnung des geförderten Volumens mit eingeht.

Bei einer Weiterbildung der Erfindung wird während der Herstellung der medizinischen Zubereitung dann, wenn sich ein Pumpenrad der Schlauchpumpe zumindest eine volle Umdrehung dreht, der Dosierfaktor der Schlauchpumpe kalibriert, vorzugsweise mit UI.

Der Dosierfaktor der Schlauchpumpe wird also nicht nur initial bei Inbetriebnahme der Anlage bestimmt, sondern wird, wenn dies möglich ist, auch während des regulären Betriebs der Anlage, also beim Herstellen von medizinischen Zubereitungen der Dosierfaktor überprüft und ggf. neu kalibriert.

Es ist insbesondere vorgesehen, dass es neben einem initialen Kalibrieren durch Bestimmen des Dosierfaktors mehrere, vorzugsweise zumindest drei, weitere Bestimmungen des Dosierfaktors während der Einsatzdauer eines Transfer-Sets gibt.

Vorzugsweise wird diese Kalibrierung im laufenden Betrieb dann durchgeführt, wenn eine hinreichende Menge universeller Flüssigkeit oder Wasser in den Zielbehälter transferiert wird, da der Flussfaktor dieser universellen Flüssigkeit immer 1,0 beträgt, so dass kein Fehler aufgrund eines unterschiedlichen Flussfaktors in die Kalibrierung eingeht. Die Kalibrierung im laufenden Betrieb erfolgt vorzugsweise bei Förderung derselben Flüssigkeit, wie diese zum initialen Bestimmen des Dosierfaktors unter Verwendung des waste bag verwendet wurde.

Besonders bevorzugt wird die Kalibrierung im laufenden Betrieb erst vorgenommen, wenn das Transfer-Set mit universeller Flüssigkeit gespült ist, der Zulauf des Zielbehälters also keine Abschnitte aufweist, in welchen sich eine andere Flüssigkeit befindet.

Da so während der gesamten Kalibrierung nur Flüssigkeit mit gleicher Dichte und gleicher Viskosität gefördert wird, wird eine höhere Genauigkeit beim Kalibrieren erreicht.

Die vorab beschriebenen erfindungsgemäßen Verfahrensschritte können umgesetzt werden durch Einrichtungen, die entsprechend ausgebildet oder geeignet sind zum Ausführen der beschriebenen Verfahrensschritte. Diese Einrichtungen können Bestandteil eines Systems sein.

Im Bereich der Erfindung liegt daher auch eine Anlage zur Herstellung einer medizinischen Zubereitung, insbesondere eine Anlage zur Herstellung von Parenteralnahrung, umfassend eine Schlauchpumpe und ein System zum Ausführen eines Verfahrens gemäß der vorstehend beschriebenen Erfindung.

Das erfindungsgemäße Verfahren ist insbesondere ausführbar mittels der erfindungsgemäßen Anlage. Die Anlage mit dem erfindungsgemäßen System ist insbesondere ausgebildet zur Ausführung des erfindungsgemäßen Verfahrens.

## Kurzbeschreibung der Zeichnungen

Der Gegenstand der Erfindung soll im Folgenden, Bezug nehmend auf die Zeichnungen Fig. 1 bis Fig. 11, anhand eines Ausführungsbeispiels erläutert werden.

Fig. 1 zeigt eine perspektivische Ansicht einer Anlage zur Herstellung einer medizinischen Zubereitung, wie sie für das erfindungsgemäße Verfahren verwendet wird.

Fig. 2 ist eine Detailansicht der Schlauchpumpe.

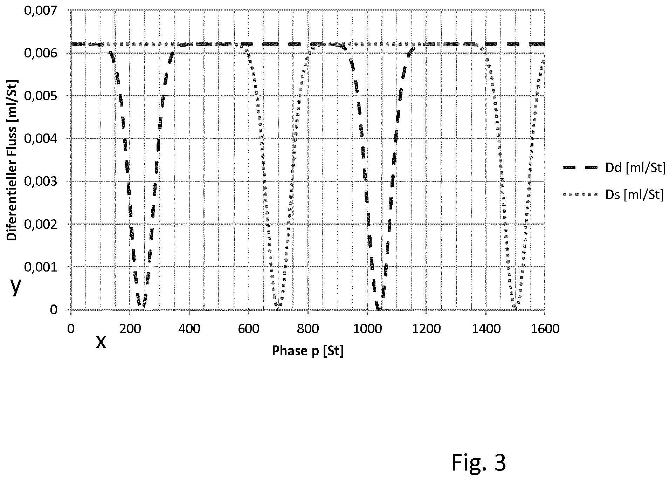

Bezugnehmend auf Fig. 3 soll die Kennlinie einer Schlauchpumpe anhand eines Ausführungsbeispiels erläutert werden.

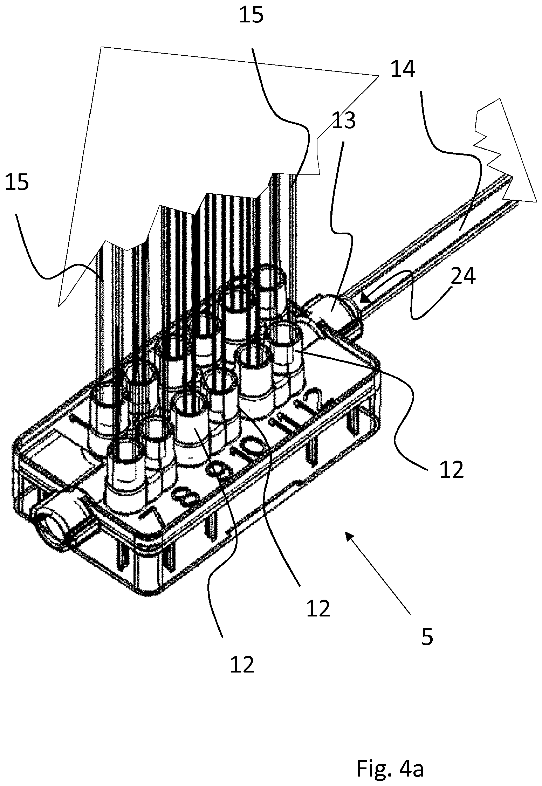

Fig. 4a bis 4c sind Detailansichten der Ventileinheit der Anlage zur Herstellung einer medizinischen Zubereitung mit deren Schläuchen.

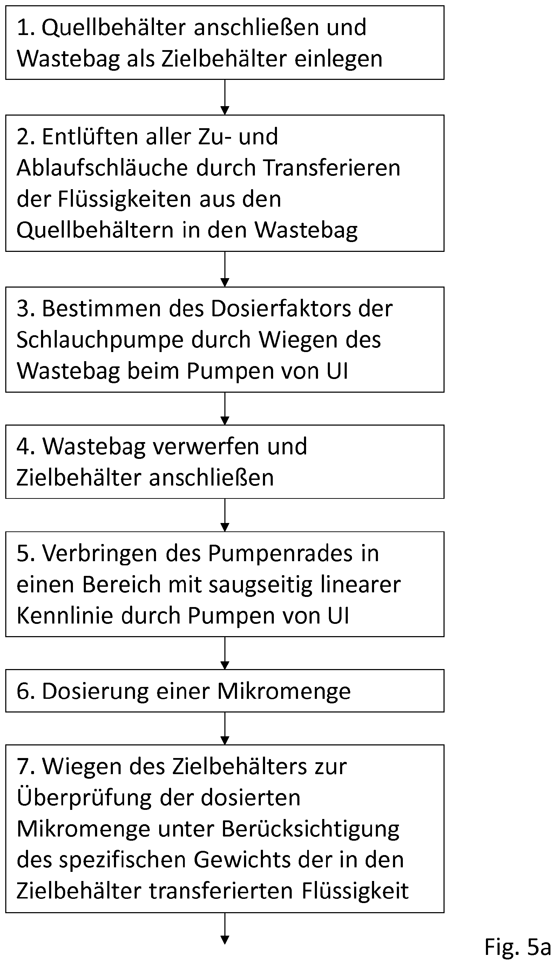

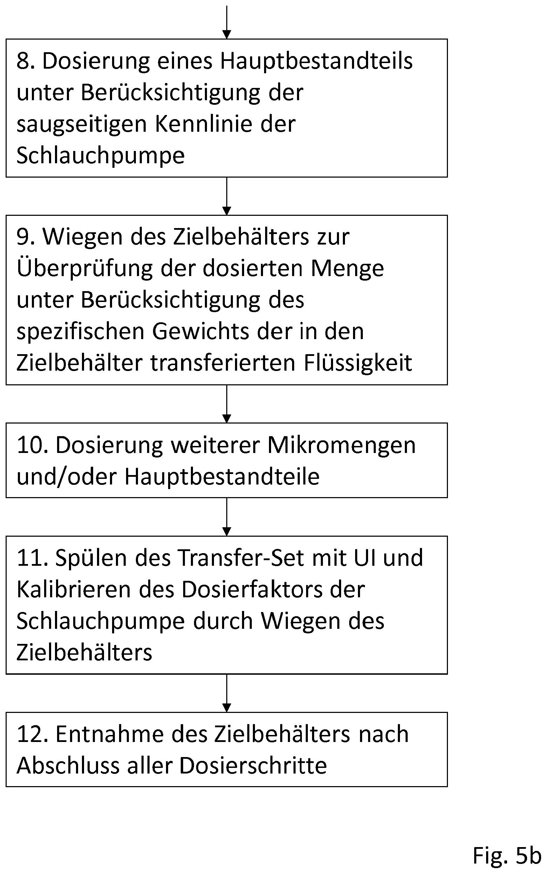

Fig. 5a und Fig. 5b zeigen anhand eines Flussdiagramms die Verfahrensschritte bei einem Ausführungsbeispiel des erfindungsgemäßen Verfahrens.

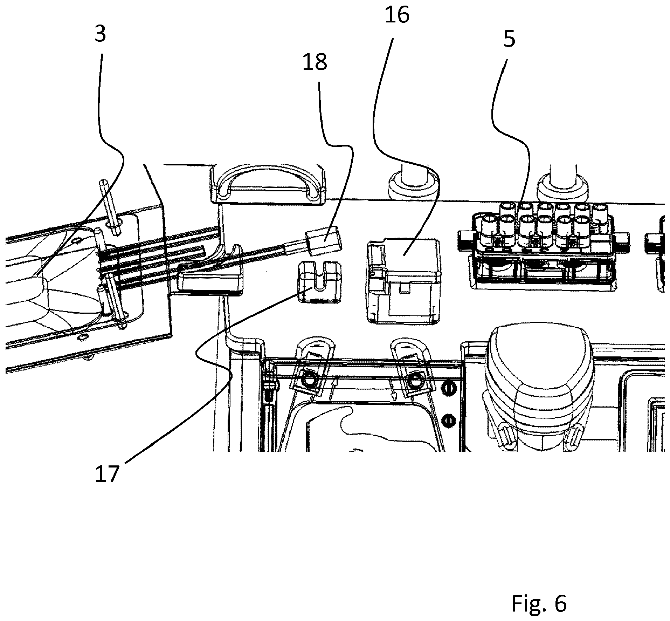

Fig. 6 ist eine Detailansicht der Anlage zur Herstellung einer medizinischen Zubereitung, in welcher Flusssensor und Blasensensor zu erkennen sind.

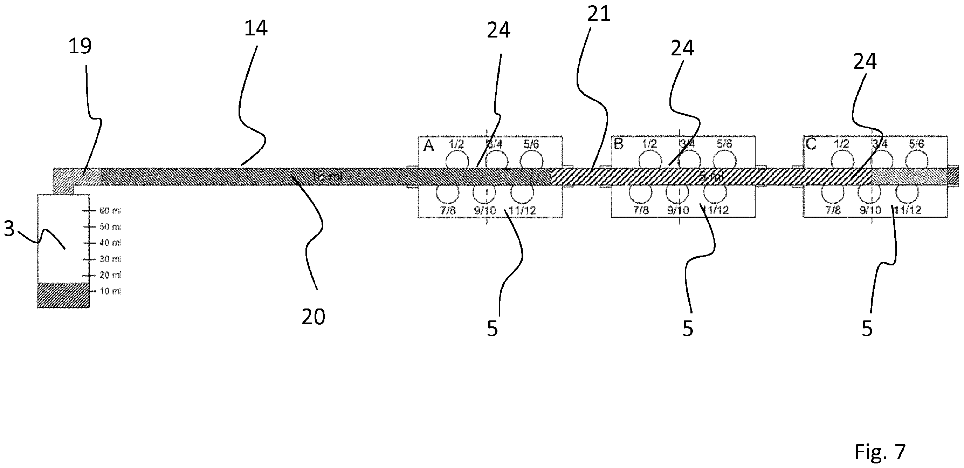

Fig. 7 ist eine schematische Darstellung des Zulaufs des Zielbehälters, anhand welcher die Berechnung der in den Zielbehälter transferierten Menge erläutert wird.

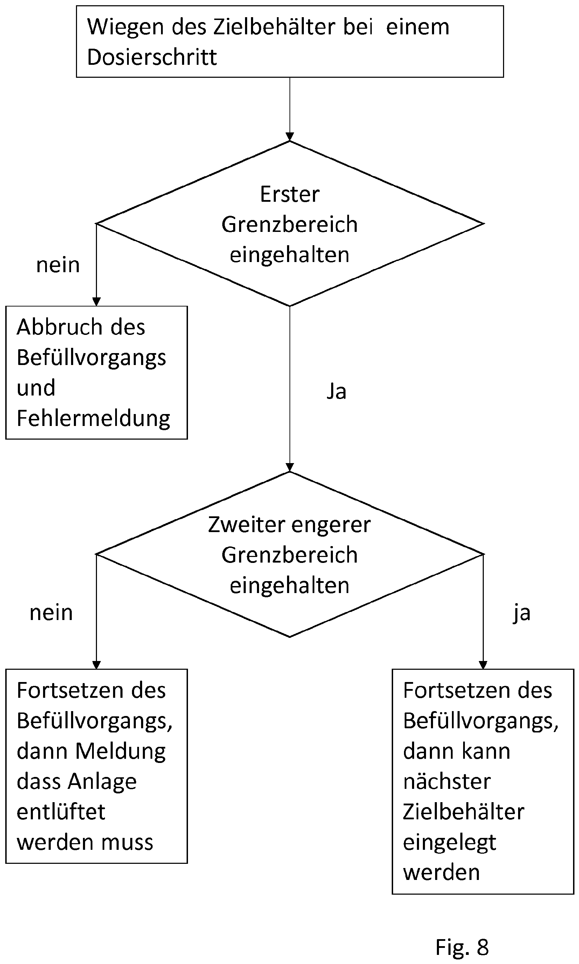

Fig. 8 ist ein Flussdiagramm, anhand dessen die Überprüfung jedes Dosierschrittes durch Wiegen des Zielbehälters erläutert wird.

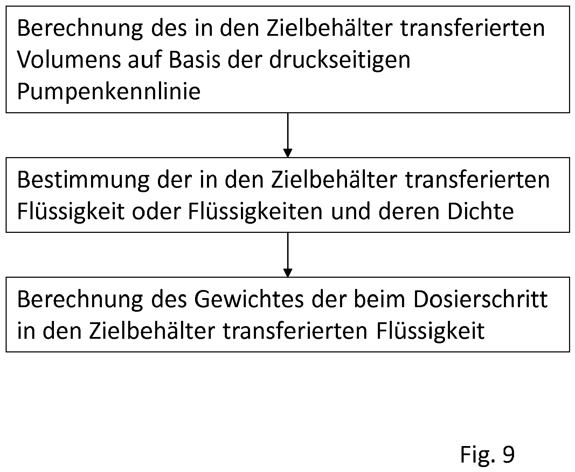

Fig. 9 ist ein Flussdiagramm, anhand dessen die Berechnung des Gewichts der in den Zielbehälter transferierten Flüssigkeit erläutert wird.

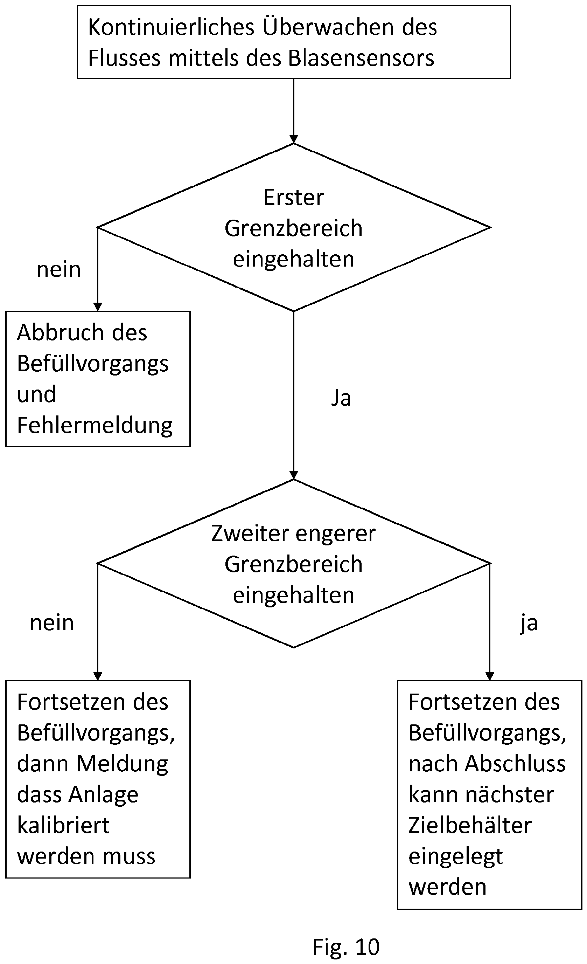

Fig. 10 ist ein Flussdiagramm, anhand dessen die Kontrolle über den Blasensensor erläutert werden soll.

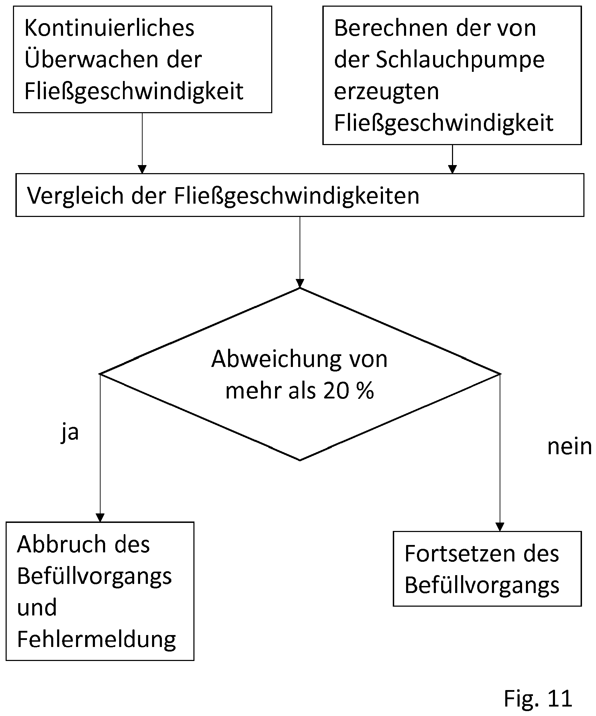

Fig. 11 ist ein Flussdiagramm, anhand dessen die Kontrolle über den Flusssensor erläutert werden soll.

Detaillierte Beschreibung der Zeichnungen

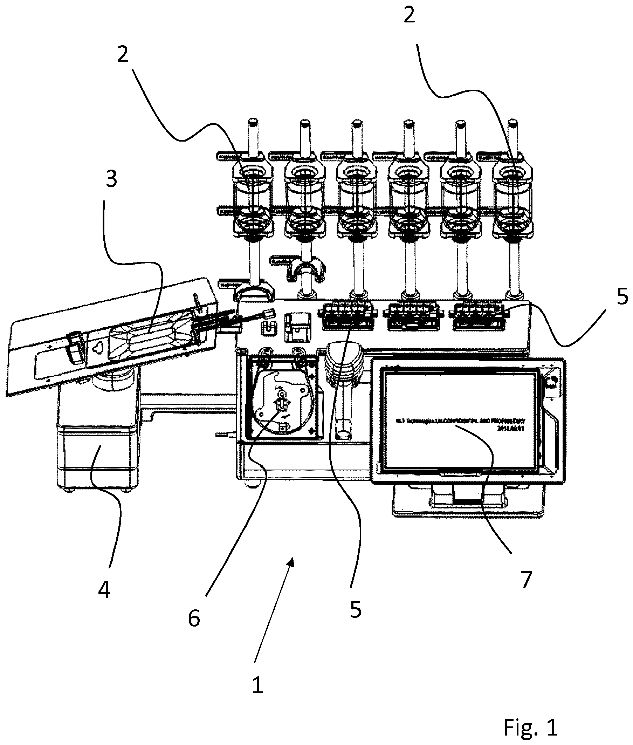

Fig. 1 zeigt eine Anlage 1 zur Herstellung einer medizinischen Zubereitung.

Die Anlage 1 zur Herstellung einer medizinischen Zubereitung umfasst eine Vielzahl von Quellbehältern 2, welche in dieser Ansicht nur teilweise dargestellt sind. Insbesondere sind in dieser Darstellung diejenigen Quellbehälter nicht dargestellt, welche die Hauptbestandteile der medizinischen Zubereitung umfassen, sowie derjenige Behälter, der mit universeller Flüssigkeit gefüllt ist. Diese Behälter können insbesondere anlagenfern, z.B. an einem an einer Schiene befestigten Haken, aufgehängt werden.

Zu erkennen ist ein als Infusionsbeutel ausgebildeter Zielbehälter 3, welcher auf einer Waage 4 angeordnet ist. Über die Waage 4 kann während des Betriebs der Anlage 1 die Menge der in den Zielbehälter 3 transferierten Flüssigkeit überprüft werden.

Um die Anlage 1 in Betrieb zu nehmen, wird ein Transfer-Set verwendet, welches eine Ventileinheit 5 sowie Schläuche umfasst, mittels der die Ventileinheit 5 zum einen mit dem Zielbehälter 3 und zum anderen mit den Quellbehältern 2 verbunden wird.

Beim Herstellen einer medizinischen Zubereitung wird über die Anlage 1 jeweils bei einem Dosierschritt ein Ventil der Ventileinheit 5 geöffnet, so dass Flüssigkeit von genau einem Quellbehälter 2 in den Zielbehälter 3 gepumpt werden kann.

Um die Flüssigkeiten zu fördern, weist die Anlage 1 hier eine einzige Schlauchpumpe 6 auf, mittels welcher Flüssigkeiten aus sämtlichen Quellbehältern 2 in den Zielbehälter 3 gepumpt werden können.

Die Anlage 1 weist ferner ein Display 7 auf, welches z.B. als Touch-Screen ausgebildet ist, mittels welchem der Benutzer die Anlage 1 programmieren und insbesondere ein Programm auswählen kann, mittels dessen ein Zielbehälter 3 mit einer vorgegebenen Zusammensetzung von Bestandteilen befüllt wird.

Die Anlage umfasst eine elektronische Steuerung (nicht dargestellt), über die die Schlauchpumpe 6 angesteuert wird und die mit der Waage 4 verbunden ist.

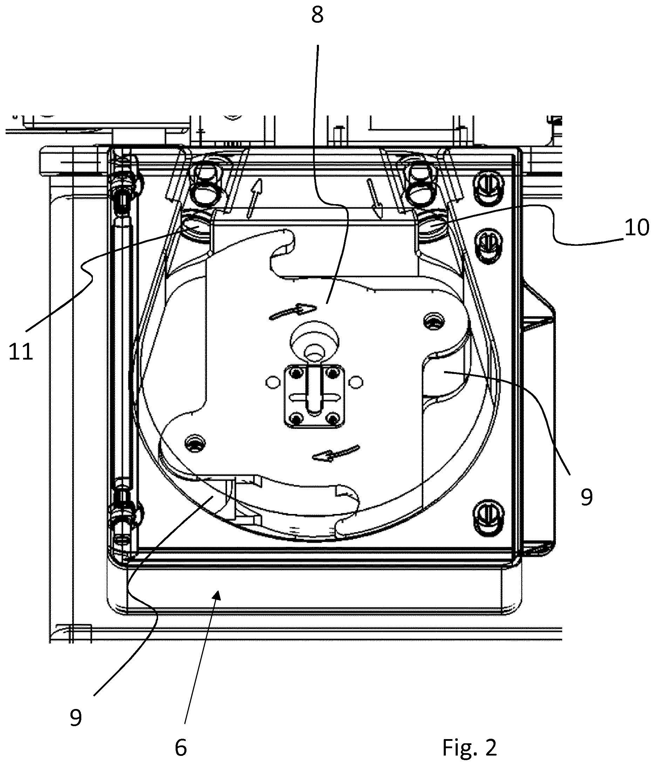

Fig. 2 ist eine Detailansicht der Schlauchpumpe 6. Diese ist hier vorzugsweise als eine Rollenpumpe bereitgestellt.

Zu erkennen ist, dass die Schlauchpumpe 6 ein Pumpenrad 8 mit zwei Rollen 9 aufweist. Der einzulegende Schlauch ist in dieser Ansicht nicht dargestellt.

Es versteht sich, dass das erfindungsgemäße Verfahren auch mit einer Schlauchpumpe mit einer anderen Anzahl an Rollen ausgeführt werden kann, insbesondere mit einer Schlauchpumpe, die drei Rollen umfasst (nicht dargestellt).

Wird ein Schlauch (nicht dargestellt) in die Schlauchpumpe 6 eingelegt, so weist die Schlauchpumpe einen Einlass 10 und einen Auslass 11 auf. In der hier dargestellten Stellung des Pumpenrads 8 sind beide Rollen 9 im Eingriff mit dem Schlauch.

Es versteht sich aber, dass, wenn die Rollen 9 sich vom Auslass 11 zum Einlass 10 bewegen, diese teils nicht im Eingriff mit dem Schlauch sind. Hieraus resultiert sowohl saugseitig, also auf Seite des Einlasses 10, als auch druckseitig, also auf Seite des Auslasses 11, eine nicht lineare Kennlinie der Pumpleistung, die Schlauchpumpe pulsiert.

Vorzugsweise beträgt die Menge der geförderten Flüssigkeit bei einer vollen Umdrehung zwischen 5 und 50 ml.

Um auch Mikromengen, also Mengen, die im unteren ml-Bereich liegen, exakt dosieren zu können, wird gemäß einem Aspekt der Erfindung die Schlauchpumpe 6 durch Drehen des Pumpenrads 8 in eine Stellung verbracht, in welcher die jeweilige Mikromenge vollständig im zumindest saugseitig linearen Bereich der Schlauchpumpe 6 dosiert werden kann.

Hierzu umfasst die Schlauchpumpe einen Drehwinkelgeber (nicht dargestellt).

In der hier darstellten Stellung des Pumpenrads 8 ist eine Rolle 9 gerade an dem Einlass 10 vorbeigestrichen und nunmehr im Eingriff mit dem eingelegten Schlauch.

Es bietet sich an, die Schlauchpumpe 6 zur Dosierung einer Mikromenge in die hier dargestellte Stellung zu bringen, um sodann die Mikromenge vollständig im Bereich der saugseitig linearen Kennlinie der Schlauchpumpe 6 dosieren zu können.

Die druckseitige und saugseitige Kennlinie ist in Fig. 3 dargestellt.

Der Phasenwinkel p ist in 1600 Einheiten eingeteilt, welche auf der x-Achse aufgetragen sind. Diese 1600 Schritte stellen eine volle Umdrehung der Pumpe dar.

Auf der y-Achse ist der differentielle Fluss, also das geförderte Volumen pro Drehwinkel-Einheit, für die Schlauchpumpe aufgetragen.

Die gestrichelte Kennlinie stellt den druckseitigen differentiellen Fluss und die gepunktete Kennlinie den saugseitigen differentiellen Fluss dar.

Zu erkennen ist, dass die Kennlinien über weite Bereiche konstant verlaufen, also Bereiche mit linearer Kennlinie vorhanden sind.

Jede Kennlinie weist allerdings zwei Einbrüche auf. Auf der Saugseite sind das die Phasenwinkel, bei denen eine der zwei Rollen neu in Eingriff geht (p=700 und p=1500). In diesen Bereichen wird das Volumen des Schlauches der Schlauchpumpe in der Nähe des saugseitigen Anschlusses verkleinert. Die Saugleistung der Pumpe ist reduziert.

Auf der Druckseite liegen die Einbrüche in den Bereichen, an denen eine Rolle aus dem Eingriff herausgeht. Dabei kehrt der Schlauch der Schlauchpumpe in seine ursprüngliche Form zurück. Der Schlauch vergrößert sein Volumen und die Förderrate der Pumpe ist druckseitig reduziert.

Für die exakte Dosierung, insbesondere einer Mikromenge, ist das geförderte Volumen der Saugseite relevant. Sämtliche Flüssigkeit, die im jeweiligen Dosierschritt dem Quellbehälter entnommen wird, gelangt letztendlich in den Zielbehälter. Es ist daher entscheidend, dass in jedem Dosierschritt das korrekte Volumen saugseitig entnommen wird.

Bei Dosierung einer sogenannten Mikromenge wird nunmehr gemäß der Erfindung bei einem Dosierschritt Flüssigkeit nur in einem der zwei linearen Bereiche der Saugseite der Pumpe gefördert.

Hierfür wird die Schlauchpumpe vor Beginn des Dosierschrittes unter Pumpen universeller Flüssigkeit vorzugsweise auf den Anfang des nächsten linearen Bereiches der Saugseite gestellt. In diesem Beispiel liegen diese Positionen etwa bei p=50 und p=850.

So lassen sich mit einer einzigen Schlauchpumpe auch Mikromengen exakt dosieren.

Die saugseitige Kennlinie der Schlauchpumpe wird vorzugsweisedazu verwendet, die Menge der dem Quellbehälter entnommenen Flüssigkeit exakter zu berechnen.

So lässt sich auch bei Dosierschritten, die im nicht linearen Bereich der Schlauchpumpe stattfinden, die saugseitige Kennlinie der Schlauchpumpe heranziehen, um die Mengen der entnommenen Flüssigkeit zu berechnen.

Es wird also bei der Berechnung berücksichtigt, dass die saugseitige Fördermenge der Schlauchpumpe nicht linear ist.

Anhand der Kennlinie Ds wird der Phasenwinkel p2 bestimmt, so dass

$$Vs = \int_{p1}^{p2} Ds(p)\,dp$$

das zu dosierende Volumen ergibt. Dabei ist p1 die Position des Pumpenrades am Beginn des Dosierschrittes und p2 die Position nach dem Dosierschritt. Die Größe Vs ist das dem Quellbehälter zu entnehmende Volumen.

Die druckseitige Kennlinie der Pumpe kann wiederum verwendet werden, um in verbesserter Weise durch Wägung des Zielbehälters zu überprüfen, ob die tatsächlich entnommene Menge der berechneten Menge entspricht.

Hierzu wird das Volumen der in den Zielbehälter eintreffenden Flüssigkeit berechnet. Weiter wird anhand der bekannten Dichte der geförderten Flüssigkeit die Masse der eintreffenden Flüssigkeit berechnet. Zur Bestimmung des Volumens der im Zielbehälter eintreffenden Flüssigkeit wird die Kennlinie Dd der Druckseite verwendet.

Die vorzugsweise durch empirische Messungen bestimmten Kennlinien können z.B. als angenäherte Formeln oder auch als einfache Wertetabelle gespeichert werden, um die saugseitige und druckseitige Pumpleistung in Abhängigkeit vom Phasenwinkel zu berechnen. Insbesondere können die Kennlinien durch Messung bestimmt und dann durch eine empirische Formel angenähert werden. Die Berechnungen in der Anlage erfolgen dann mittels der empirischen Formel oder über eine Wertetabelle.

Fig. 4a ist eine perspektivische Ansicht der für die Anlage zur Herstellung einer medizinischen Zubereitung verwendeten Ventileinheit 5.

Die Ventileinheit 5 umfasst eine Vielzahl von Zuläufen 12, welche über Schläuche 15 mit den Quellbehältern (2 in Fig. 1) verbunden werden. Über in der Ventileinheit 5 integrierte Ventile (nicht dargestellt) kann selektiv ein Schlauch 15, mittels dessen Flüssigkeit aus einem Quellbehälter entnommen wird, mit einem Schlauch 14 verbunden werden, der an dem Ablauf 13 der Ventileinheit 5 angeordnet ist.

Der Schlauch 14 weist des Weiteren einen Abschnitt auf, welcher in die Schlauchpumpe eingelegt wird.

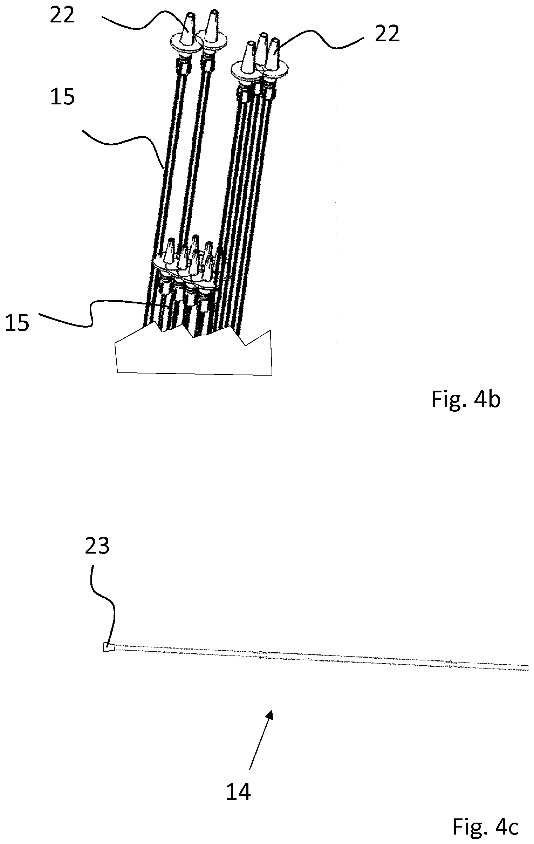

In Fig. 4b sind die Enden der Schläuche 15 zum Anschluss der Quellbehälter dargestellt. Zu erkennen sind die Anschlüsse 22 für die Quellbehälter, welche in diesem Ausführungsbespiel als Luer-Lock-Anschluss mit einem angeschlossenen Spike ausgebildet sind.

Fig. 4c zeigt den Schlauch 14, welcher den Ablauf der Ventileinheit 5 und gleichzeitig den Zulauf des Zielbehälters bildet. Zu sehen ist der Anschluss 23 für den Zielbehälter.

Die hier dargestellte Ventileinheit 5 bildet zusammen mit den Schläuchen 14, 15 und deren Anschlüssen 22, 23 das Transfer-Set, welches für den Betrieb der Anlage verwendet wird.

Dieses Transfer-Set ist vorzugsweise als Einweg-Bauteil ausgebildet und wird regelmäßig ausgetauscht. Aufgrund dieser Ausgestaltung kommen die zu transferierenden Flüssigkeiten auf dem Weg vom Quellbehälter zum Zielbehälter nur mit Komponenten des Transfer-Sets in Kontakt.

Bezugnehmend auf das Flussdiagramm gemäß Fig. 5a und Fig. 5b soll ein Ausführungsbeispiel eines erfindungsgemäßen Verfahrens zur Herstellung einer medizinischen Zubereitung erläutert werden.

Zunächst wird das zuvor beschriebene Transfer-Set verwendet, um die Quellbehälter anzuschließen. Weiter wird als Zielbehälter ein sogenannter "waste bag" eingelegt, also ein Behälter, welcher nicht dazu vorgesehen ist, bestimmungsgemäß zum Applizieren einer medizinischen Zubereitung verwendet zu werden, sondern welcher nach dem Vorbereiten der Anlage verworfen wird.

Das gesamte Transfer-Set nebst Schläuchen wird dabei mit universeller Flüssigkeit (UI), beispielsweise isotonischem Wasser, befüllt und jedes Ventil wird so lange geöffnet, dass die Schläuche (15 in Fig. 4a, b), die zu den Quellbehältern führen, gefüllt und blasenfrei sind.

Sodann kann der Dosierfaktor der Schlauchpumpe durch Wiegen des waste bag beim Pumpen von universeller Flüssigkeit bestimmt werden. Die sich aufgrund insbesondere von Toleranzen des verwendeten Schlauchs ändernde Pumpleistung der Schlauchpumpe wird nunmehr durch Bestimmen dieses Dosierfaktors kalibriert.

Der waste bag wird sodann verworfen und der erste Zielbehälter, der mit einer medizinischen Zubereitung befüllt werden soll, kann angeschlossen werden.

In diesem Ausführungsbeispiel soll zunächst eine Mikromenge in einem ersten Dosierschritt dosiert werden.

Daher wird in Schritt 5. das Pumpenrad in einen Bereich mit saugseitig linearer Kennlinie verbracht, wobei während des Verbringens des Pumpenrads in diese Stellung zunächst universelle Flüssigkeit gefördert wird.

Nunmehr kann eine Mikromenge vollständig im saugseitig linearen Bereich der Kennlinie der Pumpe dem Quellbehälter entnommen werden.

Jeder einzelne Dosierschritt, also auch der Schritt der Dosierung einer Mikromenge, wird durch Wiegen des Zielbehälters überprüft.

Dabei wird die Dichte der in den Zielbehälter transferierten Flüssigkeit berücksichtigt, indem berechnet wird, welche Flüssigkeit bzw. welche Flüssigkeiten sich beim Entnehmen der Mikromenge in Schritt 5. im Zulauf des Zielbehälters befinden und in diesen transferiert werden.

Weiter wird bei der Überprüfung durch Wägung auch unter Berücksichtigung der druckseitigen Kennlinie der Schlauchpumpe möglichst exakt berechnet, welches Volumen bei dem jeweiligen Dosierschritt in den Zielbehälter transferiert wurde. Dieses Volumen stimmt aufgrund der phasenverschobenen Kennlinien von Saugseite und Druckseite nicht immer überein.

Sodann wird ein Hauptbestandteil der medizinischen Zubereitung unter Berücksichtigung der saugseitigen Kennlinie der Schlauchpumpe dosiert. Im Unterschied zur Dosierung von Mikromengen wird bei der Dosierung der Hauptbestandteile die Schlauchpumpe auch im nicht linearen Bereich betrieben.

Bei der Berechnung der dem Quellbehälter entnommenen Menge des jeweiligen Hauptbestandteils wird die saugseitige Kennlinie der Schlauchpumpe aber berücksichtigt, um das saugseitig entnommene Volumen genau vorhersagen zu können.

Auch die Überprüfung der dem Quellbehälter für einen Hauptbestandteil entnommenen Menge wird unter Berücksichtigung der Dichte der in den Zielbehälter transferierten Flüssigkeit sowie unter Berücksichtigung der druckseitigen Kennlinie der Schlauchpumpe vorgenommen.

Sowohl beim Dosieren von Mikromengen als auch beim Dosieren von Hauptbestandteilen geht als weiterer Faktor in die Berechnung des Volumens der geförderten Flüssigkeit vorzugsweise auch ein Flussfaktor ein, welcher von der Art, insbesondere von der Viskosität, der geförderten Flüssigkeit abhängig ist. Wasser wird ein Flussfaktor von 1,0 zugeordnet, bei zähflüssigen Komponenten wie Glukoselösungen ändert sich der Flussfaktor deutlich.

Es hat sich gezeigt, dass eine pauschale Berücksichtigung des Flussfaktors in Abhängigkeit der bei jedem Dosierschritt entnommenen Flüssigkeit ausreichend ist, da ein viskositätsbedingter Einfluss der Pumpleistung in erster Linie aufgrund der am Anschluss des Quellbehälters vorhandenen Engstelle (z.B. Spike) vorhanden ist.

Das dem Zielbehälter bei einem Dosierschritt hinzufügte Gewicht kann im Detail wie folgt berechnet werden:

$$F * Vs = \int_{p1}^{p2} Ds(p)\,dp$$

Vs ist das bei einem Dosierschritt zu dosierende Volumen. Dieses entspricht dem Volumen der Saugseite, an welcher ein Quellbehälter angeschlossen ist.

p1 ist die Position des Pumpenrads vor dem Dosierschritt, insbesondere die Endstellung eines vorherigen Dosierschrittes oder der Beginn des linearen Bereiches, in welchen das Pumpenrad vorher gedreht wurde.

p2 ist die berechnete Position des Pumpenrads nach dem Dosierschritt, also das Ergebnis der Berechnung für den Drehwinkel der Pumpe bei dem Dosierschritt.

F ist der Flussfaktor, also der Korrekturfaktor für die jeweilige Viskosität des Mediums.

Ds(p) ist Kennlinie der Saugseite (Konstante) und p die Phase des Pumpenrades.

Die Phasen p1 und p2 können sich dabei um mehrere Umdrehungen unterscheiden.

Der Flussfaktor F ist also eine Korrektur für einen zusätzlichen Schlupf der Pumpe durch gegenüber Wasser erhöhte Viskosität. Das zu dosierende Volumen wird insbesondere um den Faktor F höher angesetzt als bei Wasser.

Nahezu alle für eine medizinische Zubereitung verwendeten Medien haben die gleiche oder eine höhere Viskosität als Wasser. Medien mit einer geringeren Viskosität sind sehr selten. Folglich gilt in der Regel F >= 1.

Das auf der Druckseite erwartete Volumen, anhand welchem das Gewicht der bei einem Dosierschritt in den Zielbehälter geförderten Flüssigkeitsmenge berechnet wird, beträgt:

$$Vd = \int_{p1}^{p2} Dd(p)\,dp$$

Dieses berechnete Gewicht dient der Überprüfung des jeweiligen Dosierschritts über die Wägung.

Vd ist das auf der Druckseite erwartete Volumen, also das Volumen an Flüssigkeit, welches bei dem Dosierschritt in den auf der Waage befindlichen Zielbehälter gefördert wird.

Dd(p)ist Kennlinie der Druckseite. Der Flussfaktor F geht in die Berechnung des druckseitig geförderten Volumens nicht ein, da der "Schlupf" der Pumpe ja nicht gefördert wird.

Der erwartete Massezuwachs G auf der Waage lautet dann:

$$G = Vd * \rho$$

mit der Dichte $\rho$ des geförderten Mediums.

$\rho$ ist also das spezifische Gewicht der bei einem Dosierschritt in den Zielbehälter transferierten Flüssigkeit, also zunächst der Flüssigkeit, die im Zulauf des Zielbehälters bereits vorhanden ist. Werden mehrere verschiedene Flüssigkeiten während eines Dosierschritts in den Zielbehälter transferiert, so wird das spezifische Gewicht der Flüssigkeiten entsprechend ihrer Menge ins Verhältnis gesetzt.

Als nächster Schritt werden weitere Mikromengen oder weitere Hauptbestandteile in weiteren Dosierschritten zugeführt. Die Schritte 5. bis 9. können also wiederholt werden, bis alle gewünschten Bestandteile im Zielbehälter sind.

Es versteht sich, dass die Schritte 5. bis 7., also die Dosierung einer Mikromenge, sowie die Schritte 8. und 9., also die Dosierung eines Hauptbestandteils, auch austauschbar sind, also in unterschiedlicher Reihenfolge vorgenommen werden können.

Zum Ende jedes Befüllvorgangs wird das Transfer-Set mit universeller Flüssigkeit gespült und ggf. die gewünschte Restmenge universeller Flüssigkeit dem Zielbehälter zugeführt.

Es ist vorgesehen, beispielsweise diese Spülphase, bei welcher sich das Pumpenrad der Schlauchpumpe um mehr als eine volle Umdrehung dreht, zu nutzen, um im laufenden Betrieb den Dosierfaktor der Schlauchpumpe neu zu bestimmen, indem der Zielbehälter gewogen wird. Der Dosierfaktor kann somit im laufenden Betrieb nachkalibriert werden. Dieser kann sich beispielsweise dadurch ändern, dass sich die Elastizität und Form des in die Schlauchpumpe eingelegten Schlauchs ändert.

Nach Abschluss aller Dosierschritte und dem Spülen des Transfer-Sets kann der Zielbehälter entnommen werden und ein neuer Zielbehälter angeschlossen werden.

Es versteht sich, dass sämtliche hier dargestellten Schritte mit Ausnahme des Anschließens der Quell- und Zielbehälter und des Startens der Anlage vorzugsweise automatisiert ablaufen.

Fig. 6 ist eine weitere Detaildarstellung der Fig. 1. Zu erkennen ist wiederum der Zielbehälter 3. Weiter zu erkennen ist eine Ventileinheit 5.

Der hier nicht dargestellte Schlauch, welcher die Ventileinheit 5 mit dem Zielbehälter 3 verbindet, und der insbesondere in die Schlauchpumpe eingelegt wird, wird zunächst in einen Flusssensor 16 eingelegt.

Über den Flusssensor 16 wird der saugseitige Durchfluss im Schlauch gemessen und die Förderleistung der Schlauchpumpe kann so auf Plausibilität überprüft werden. Kommt es beispielsweise im Bereich der Ventileinheit oder am Anschluss eines Quellbehälters zu einer Verstopfung, so wird sich der saugseitige Durchfluss derartig verringern, dass mittels des Flusssensors 16 ein Fehler erfasst werden kann. Insbesondere bei Dosierung einer Mikromenge wird sich der Schlauch auch im Bereich des Flusssensors 16 zunächst zusammenziehen, was zur Folge hat, dass der detektierte Durchfluss verringert und auf eine Verstopfung geschlossen werden kann. Über die elektronische Steuerung kann sodann eine Fehlermeldung generiert und dem Benutzer angezeigt werden.

Der Flusssensor 16 ist vorzugsweise als Ultraschallsensor ausgebildet. Insbesondere bei niedrigen Fließgeschwindigkeiten ist ein derartiger Sensor in der Regel nicht genau genug, um allein über den Flusssensor die Menge der saugseitig geförderten Flüssigkeit hinreichend genau bestimmen zu können.

Vorzugsweise wird der Flusssensor daher allein zur Kontrolle derart verwendet, dass bei Überschreitung eines Schwellwertes der Differenz von berechneter Förderleistung der Schlauchpumpe und daraus resultierender berechneter Durchflussmenge gegenüber der vom Flusssensor bestimmen Durchflussmenge ein Fehler angenommen wird.

Drucksseitig wird der Schlauch in einen Blasensensor 17 eingelegt. Es handelt sich dabei um einen Ultraschall-Sensor, welcher Blasen erfasst und ab einem gewissen Schwellwert die Anlage abschaltet und dem Benutzer einen Fehler anzeigt.

Fig. 7 ist eine schematische Darstellung des Schlauchs 14, welcher die Ventileinheit 5 mit dem Zielbehälter 3 verbindet. In diesem Ausführungsbeispiel sind drei hintereinander geschaltete Ventileinheiten dargestellt, was aber auf das Grundprinzip keinen Einfluss hat. Die hier dargestellten drei Ventileinheiten 5 können genauso gut zu einer einzigen Ventileinheit zusammengefasst sein.

Mittels der Ventileinheit 5 wird bei jedem Dosierschritt der Zulauf zu einem Quellbehälter geöffnet, so dass Flüssigkeit aus dem Quellbehälter über das jeweilige Ventil der Ventileinheit zunächst in die Ventileinheit und sodann in den Schlauch 14 treten kann.

Der Schlauch 14 sowie die Sammelkanäle 22 der Ventileinheiten 5 bilden ein Volumen, in welches die den jeweiligen Quellbehältern entnommene Flüssigkeit zunächst transferiert wird.

Zur Berechnung des Gewichts der Flüssigkeit, welche bei einem Dosierschritt im Zielbehälter ankommt, wird daher nicht die Dichte der bei dem jeweiligen Dosierschritt entnommenen Flüssigkeit zugrunde gelegt. Vielmehr werden der Schlauch 14 und die Sammelkanäle 24 der Ventileinheit(en) 5 derart betrachtet, dass sich verschiedene Flüssigkeiten, nämlich eine erste Flüssigkeit 19, eine zweite Flüssigkeit 20 und eine dritte Flüssigkeit 21 in unterschiedlichen Abschnitten des Schlauchs 14 und/oder des anschließenden Sammelkanal 24 befinden.

Wird beispielsweise eine Mikromenge dosiert, wird zunächst das spezifische Gewicht der ersten Flüssigkeit 19 zugrunde gelegt.

Durch diesen theoretischen "material stack" ("Materialanordnung") kann die Genauigkeit der Überprüfung verbessert werden. Insbesondere ist es möglich, jeden einzelnen Dosierschritt zu überprüfen und zu bewerten.

Fig. 8 ist ein Flussdiagramm, anhand dessen die Überprüfung jedes Dosierschrittes durch Wiegen des Zielbehälters erläutert wird.

Bei jedem Dosierschritt wird das in den Zielbehälter transferierte Gewicht als ein Sollgewicht berechnet. Dies erfolgt, wie vorstehend beschrieben, auf Basis der druckseitigen Kennlinie der Schlauchpumpe und des spezifischen Gewichts der in den Zielbehälter transferierten Flüssigkeit.

Weicht beim Wiegen des Zielbehälters das durch die Wägung bestimmte Gewicht derart vom errechneten Gewicht ab, dass ein erster Grenzbereich nicht eingehalten wird, der die Qualität der medizinischen Zubereitung beeinträchtigen würde oder der auf einen Fehler schließen lässt, wird der Befüllvorgang abgebrochen und es erfolgt eine Fehlermeldung. Der Benutzer kann dann ggf. den Fehler beseitigen, einen waste bag einlegen und die Anlage neu kalibrieren.

Ansonsten wird der Befüllvorgang fortgesetzt.

Liegt das mittels der Wägung bestimmte Gewicht nicht innerhalb eines zweiten engeren Grenzbereiches, welcher zwar z.B. auf eine nicht hinreichende Kalibrierung der Anlage schließen lässt, welcher aber auf eine so geringe Abweichung der dosierten Menge schließen lässt, dass sie die Qualität der medizinischen Zubereitung nicht beeinträchtigt, wird der Befüllvorgang fortgesetzt.

Nach Abschluss des Befüllvorgangs erhält der Benutzer der Anlage aber eine Meldung, dass die Anlage kalibriert werden muss.

Ansonsten kann nach Abschluss des Befüllvorgangs der nächste Zielbehälter eingelegt werden.

Fig. 9 ist ein Flussdiagramm, anhand dessen die Berechnung des Sollgewichts bei einem Dosierschritt erläutert wird.

Das Volumen der eingeleiteten Flüssigkeit wird anhand der druckseitigen Kennlinie der Schlauchpumpe berechnet.

Sodann wird bestimmt, welche Flüssigkeit oder welche Flüssigkeiten bei dem Dosierschritt in den Zielbehälter gelangt ist bzw. sind. Dies erfolgt wie unter Bezugnahme auf Fig. 7 beschrieben wurde.

Über das spezifische Gewicht der transferierten Flüssigkeit oder der Flüssigkeiten kann sodann das Sollgewicht berechnet werden.

Dieses Sollgewicht dient der Bestimmung der in Fig. 8 genannten Grenzwerte. So könnte beispielsweise ein erster Grenzbereich als Abweichung von über 10% und ein zweiter Grenzbereich als Abweichung über 5% definiert werden.

Es versteht sich, dass die Grenzbereiche auch in Abhängigkeit der bei einem Dosierschritt entnommenen Flüssigkeit variiert werden können, da es Bestandteile gibt, bei denen Abweichungen in der Menge mehr oder weniger kritisch für die Qualität der medizinischen Zubereitung sind.

Fig. 10 ist ein Flussdiagramm, anhand dessen die Kontrolle über den Blasensensor erläutert werden soll.

Über den nach der Schlauchpumpe angeordneten Blasensensor wird laufend die Blasenmenge in der transferierten Flüssigkeit überwacht.

In diesem Ausführungsbespiel sind auch zwei Grenzbereiche vorgesehen.

Liegt die Blasenmenge in einem Grenzbereich, der für die Qualität des hergestellten Produktes nicht akzeptabel ist, wird der Befüllvorgang unterbrochen und es erfolgt eine Fehlermeldung.

Wird ein zweiter, engerer Grenzbereich nicht eingehalten, kann zwar der Befüllvorgang fortgesetzt und der Zielbehälter bestimmungsgemäß verwendet werden, es erfolgt aber mit Abschluss des Befüllvorgangs eine Fehlermeldung, dass die Anlage entlüftet werden muss.

Ansonsten kann nach Abschluss des Befüllvorgangs der nächste Zielbehälter eingelegt werden

Fig. 11 ist ein Flussdiagramm, anhand dessen die Kontrolle über den Flusssensor erläutert werden soll.

Die Fließgeschwindigkeit wird laufend, vorzugsweise auf Basis der saugseitigen Kennlinie der Schlauchpumpe, berechnet.

Parallel dazu wird die Fließgeschwindigkeit mit einem strömungsseitig vor der Schlauchpumpe angeordneten Flusssensor gemessen.

Gemessene und berechnete Fließgeschwindigkeit werden verglichen.

Liegt eine Abweichung über einem Schwellwert, in diesem Beispiel 20%, vor, wird auf einen Fehler (z.B. Okklusion) geschlossen und der Befüllvorgang wird abgebrochen.

Über eine Fehlermeldung wird der Benutzer informiert.

Um den Fehler besser lokalisieren zu können, wird vorzugsweise bei jeglicher Fehlermeldung dem Benutzer der Quellbehälter angezeigt (z.B. über eine Nummer auf einem Bildschirm), aus welchem Flüssigkeit bei Fehlereintritt entnommen wurde.

Durch die Erfindung kann die Genauigkeit bei der Herstellung einer medizinischen Zubereitung unter Verwendung einer Schlauchpumpe verbessert werden.

Bezugszeichenliste

1 Anlage
2 Quellbehälter
3 Zielbehälter
4 Waage
5 Ventileinheit
6 Schlauchpumpe
7 Display
8 Pumpenrad
9 Rolle
10 Einlass
11 Auslass
12 Zulauf
13 Ablauf
14 Schlauch
15 Schlauch
16 Flusssensor
17 Blasensensor
18 Anschluss
19 erste Flüssigkeit
20 zweite Flüssigkeit
21 dritte Flüssigkeit
22 Anschluss
23 Anschluss
24 Sammelkanal

**Patentansprüche**

1. Verfahren zur Herstellung einer medizinischen Zubereitung, insbesondere zur parenteralen Ernährung, wobei aus einer Mehrzahl von Quellbehältern (2) Flüssigkeiten mit einer Schlauchpumpe (6) in einen Zielbehälter (3) transferiert werden, wobei die Schlauchpumpe (6) zumindest einen Bereich mit linearer und einen Bereich mit nicht linearer Kennlinie der Pumpleistung aufweist, **dadurch gekennzeichnet, dass** zur Dosierung aus zumindest einem Quellbehälter (2) die Schlauchpumpe (6) in eine Stellung gebracht wird, so dass die gesamte Dosierung aus dem Quellbehälter (2) in dem Bereich mit der linearen Kennlinie der Pumpleistung erfolgt.

2. Verfahren zur Herstellung einer medizinischen Zubereitung nach dem vorstehenden Anspruch, **dadurch gekennzeichnet, dass** die Schlauchpumpe (6) in eine Stellung gebracht wird, in welcher die saugseitige Kennlinie der Schlauchpumpe (6) linear ist.

3. Verfahren zu Herstellung einer medizinischen Zubereitung nach einem der vorstehenden Ansprüche, **dadurch gekennzeichnet, dass**, um die Schlauchpumpe (6) in die gewünschte Stellung mit linearer Kennlinie zu bringen, Flüssigkeit aus einem anderen Quellbehälter entnommen wird, als aus dem dosiert werden soll, insbesondere aus einem Quellbehälter mit universeller Flüssigkeit oder Wasser.

4. Verfahren zur Herstellung einer medizinischen Zubereitung nach einem der vorstehenden Ansprüche, **dadurch gekennzeichnet, dass** im Bereich der linearen Kennlinie eine Kleinstmenge mit einem Volumen von unter 10 ml, bevorzugt unter 5 ml, besonders bevorzugt kleiner oder gleich 3 ml, gefördert wird.

5. Verfahren zur Herstellung einer medizinischen Zubereitung nach einem der vorstehenden Ansprüche, **dadurch gekennzeichnet, dass** für das Transferieren der Flüssigkeiten aus allen Quellbehältern (2) in den Zielbehälter (3) genau eine einzige Schlauchpumpe verwendet wird.

6. Verfahren zur Herstellung einer medizinischen Zubereitung nach einem der vorstehenden Ansprüche, **dadurch gekennzeichnet, dass** in zumindest einem weiteren Dosierschritt eine Menge mit einem Volumen von über 15 ml, vorzugsweise über 20 ml gefördert wird, wobei die Schlauchpumpe sowohl im Bereich mit linearer Kennlinie als auch in einem Bereich mit nichtlinearer Kennlinie betrieben wird.

7. Verfahren zur Herstellung einer medizinischen Zubereitung nach einem der vorstehenden Ansprüche, **dadurch gekennzeichnet, dass** anhand der dem jeweiligen Quellbehälter zu entnehmenden Menge an Flüssigkeit die hierfür erforderliche Drehung eines Pumpenrades auf Basis der saugseitigen Kennlinie der Schlauchpumpe berechnet wird.

8. Verfahren zur Herstellung einer medizinischen Zubereitung nach einem der vorstehenden Ansprüche, **dadurch gekennzeichnet, dass** bei jedem Dosierschritt der Zielbehälter (3) gewogen wird und so die Menge der jeweils transferierten Flüssigkeit überprüft wird.

9. Verfahren zur Herstellung einer medizinischen Zubereitung nach einem der vorstehenden Ansprüche, **dadurch gekennzeichnet, dass** die Menge der von der Schlauchpumpe (6) geförderten Flüssigkeit berechnet wird und der Zielbehälter (3) gewogen wird, um die Menge der geförderten Flüssigkeit zu überprüfen, wobei die Abfolge verschiedener Flüssigkeiten in einem Zulauf des Zielbehälters (3) berücksichtigt wird, um bei der Überprüfung beim Wiegen die spezifische Masse der Flüssigkeit einzuberechnen.

10. Verfahren zur Herstellung einer medizinischen Zubereitung nach einem der vorstehenden Ansprüche, **dadurch gekennzeichnet, dass** der Zielbehälter (3) bei jedem einzelnen Dosierschritt gewogen und so die Menge der in den Zielbehälter (3) transferierten Flüssigkeit bei jedem einzelnen Dosierschritt überprüft wird.

11. Verfahren zur Herstellung einer medizinischen Zubereitung nach einem der vorstehenden Ansprüche, **dadurch gekennzeichnet, dass** die Menge der bei einem Dosierschritt in den Zielbehälter (3) transferierten Flüssigkeit unter Berücksichtigung der druckseitigen Kennlinie der Schlauchpumpe (6) berechnet wird.

12. Verfahren zur Herstellung einer medizinischen Zubereitung nach einem der vorstehenden Ansprüche, **dadurch gekennzeichnet, dass** mit einem Flusssensor (16) die Förderleistung der Schlauchpumpe (6) überprüft wird und/oder dass mit einem Blasensensor (17) überprüft wird, dass sich keine Blasen in einem Zulauf zum Zielbehälter (3) befinden.

13. Verfahren zur Herstellung einer medizinischen Zubereitung nach einem der vorstehenden Ansprüche, **dadurch gekennzeichnet, dass** ein Dosierfaktor der Schlauchpumpe (6) in einem vorgeschalteten Kalibrierschritt mittels Wiegen eines Zielbehälters (3) bestimmt wird und/oder dass bei einem Dosierschritt aus einem Quellbehälter, bei welchem sich ein Pumpenrad (8) der Schlauchpumpe (6) zumindest eine volle Umdrehung dreht, der Dosierfaktor der Pumpe kalibriert wird.

14. Verfahren zur Herstellung einer medizinischen Zubereitung nach einem der vorstehenden Ansprüche, **dadurch gekennzeichnet, dass** zum Transferieren der Flüssigkeiten aus den Quellbehältern (2) in den Zielbehälter (3) ein Transfer-Set verwendet wird, welches eine Ventileinheit (5), einen Schlauch (14), der in die Schlauchpumpe einlegbar ist, und eine Mehrzahl von Schläuchen (15) zum Anschluss der Quellbehälter (2) umfasst.

15. Anlage zur Herstellung einer medizinischen Zubereitung, insbesondere Anlage zur Herstellung von Parenteralnahrung, umfassend eine Schlauchpumpe und **gekennzeichnet durch** ein System mit einer elektronischen Steuerung zum Ausführen eines Verfahrens nach einem der vorstehenden Ansprüche 1 bis 14.

**Claims**

1. A method for producing a medical preparation, in particular for parenteral nutrition, wherein liquids are transferred using a hose pump (6) from a plurality of source containers (2) into a target container (3), wherein the hose pump (6) has at least one region with a linear characteristic curve and one region with a non-linear characteristic curve of the pump output, **characterized in that**, in order to meter from at least one source container (2), the hose pump (6) is brought to a position, such that the metering from the source container (2) takes place entirely in the region with the linear characteristic curve of the pump output.

2. The method for producing a medical preparation according to the preceding claim, **characterized in that** the hose pump (6) is brought to a position in which the suction-side characteristic curve of the hose pump (6) is linear.

3. The method for producing a medical preparation according to one of the preceding claims, **characterized in that**, in order to bring the hose pump (6) to the desired position with a linear characteristic curve, liquid is removed from a source container other than that from which metering is to be carried out, in particular from a source container with universal liquid or water.

4. The method for producing a medical preparation according to one of the preceding claims, **characterized in that** a very small quantity with a volume of under 10 ml, preferably under 5 ml, particularly preferably less than or equal to 3 ml, is delivered in the region of the linear characteristic curve.

5. The method for producing a medical preparation according to one of the preceding claims, **characterized in that** precisely one single hose pump is used for transferring the liquids from all of the source containers (2) into the target container (3).

6. The method for producing a medical preparation according to one of the preceding claims, **characterized in that**, in at least one further metering step, a quantity with a volume of over 15 ml, preferably of over 20 ml, is delivered, wherein the hose pump is operated both in the region with the linear characteristic curve and also in the region with a non-linear characteristic curve.

7. The method for producing a medical preparation according to one of the preceding claims, **characterized in that**, using the quantity of liquid that is to be removed from the respective source container, the rotation of an impeller required therefor is calculated on the basis of the suction-side characteristic curve of the hose pump.

8. The method for producing a medical preparation according to one of the preceding claims, **characterized in that**, during each metering step, the target container (3) is weighed and the quantity of the respectively transferred liquid is thus checked.

**9.** The method for producing a medical preparation according to one of the preceding claims, **characterized in that** the quantity of the liquid delivered by the hose pump (6) is calculated and the target container (3) is weighed in order to check the quantity of the liquid delivered, wherein the sequence of different liquids in an inflow of the target container (3) is taken into consideration in order to factor in the specific mass of the liquid during the checking by weighing.

**10.** The method for producing a medical preparation according to one of the preceding claims, **characterized in that** the target container (3) is weighed during each individual metering step, and the quantity of the liquid transferred into the target container (3) is thus checked during each individual metering step.

**11.** The method for producing a medical preparation according to one of the preceding claims, **characterized in that** the quantity of the liquid transferred into the target container (3) in one metering step is calculated taking into account the pressure-side characteristic curve of the hose pump (6).

**12.** The method for producing a medical preparation according to one of the preceding claims, **characterized in that** the delivery rate of the hose pump (6) is checked with a flow sensor (16) and/or **in that** a bubble sensor (17) is used to check that there are no bubbles in an inflow to the target container (3).

**13.** The method for producing a medical preparation according to one of the preceding claims, **characterized in that** a metering factor of the hose pump (6) is determined in a preceding calibration step by weighing a target container (3) and/or **in that** the metering factor of the pump is calibrated in a metering step from a source container in which an impeller (8) of the hose pump (6) rotates for at least one full revolution.

**14.** The method for producing a medical preparation according to one of the preceding claims, **characterized in that**, in order to transfer the liquids from the source containers (2) into the target container (3), a transfer set is used which comprises a valve unit (5), a hose (14), which is insertable into the hose pump, and a plurality of hoses (15) for connecting the source containers (2).

**15.** An installation for producing a medical preparation, in particular an installation for producing parenteral nutrition, comprising a hose pump and **characterized in that** it has a system with an electronic controller for carrying out a method according to one of the preceding claims 1 to 14.

**Revendications**

**1.** Procédé de fabrication d'une préparation médicamenteuse, notamment pour l'alimentation parentérale, dans lequel des liquides sont transférés d'une pluralité de récipients sources (2) dans un récipient cible (3) au moyen d'une pompe tubulaire (6), dans lequel la pompe tubulaire (6) présente au moins une zone avec une courbe caractéristique linéaire et une zone avec une courbe caractéristique non linéaire de la puissance de pompage, **caractérisé en ce que**, pour le dosage à partir d'au moins un récipient source (2), la pompe tubulaire (6) est amenée dans une position, de sorte que la totalité du dosage à partir du récipient source (2) s'effectue dans la zone présentant la caractéristique linéaire de la puissance de pompage.

**2.** Procédé de fabrication d'une préparation médicamenteuse selon la revendication précédente, **caractérisé en ce que** la pompe tubulaire (6) est amenée dans une position, dans laquelle la courbe caractéristique côté aspiration de la pompe tubulaire (6) est linéaire.

**3.** Procédé de fabrication d'une préparation médicamenteuse selon l'une des revendications précédentes, **caractérisé en ce que**, pour amener la pompe tubulaire (6) dans la position souhaitée avec une courbe caractéristique linéaire, le liquide est prélevé dans un autre récipient source, que celui à partir duquel le dosage doit être effectué, notamment à partir d'un récipient source contenant un liquide universel ou de l'eau.

**4.** Procédé de fabrication d'une préparation médicamenteuse selon l'une des revendications précédentes, **caractérisé en ce que**, dans la zone de la courbe caractéristique linéaire, une quantité minime d'un volume inférieur à 10 ml, préférablement inférieur à 5 ml, en particulier préférablement inférieur ou égal à 3 ml, est acheminée.

**5.** Procédé de fabrication d'une préparation médicamenteuse selon l'une des revendications précédentes, **caractérisé en ce que** précisément une seule pompe tubulaire est utilisée pour transférer les liquides de tous les récipients sources (2) dans le récipient cible (3).

**6.** Procédé de fabrication d'une préparation médicamenteuse selon l'une des revendications précédentes, **caractérisé en ce que**, dans au moins une autre étape de dosage, une quantité d'un volume supérieur à 15 ml, de préférence supérieur à 20 ml, est acheminée, la pompe tubulaire fonctionnant à la fois dans la zone à caractéristique linéaire et dans une zone à caractéristique non linéaire.

**7.** Procédé de fabrication d'une préparation médicamenteuse selon l'une des revendications précédentes, **caractérisé en ce que**, à l'aide de la quantité de liquide à prélever du récipient source respectif, la rotation d'une roue de pompe nécessaire à cet effet est calculée sur la base de la courbe caractéristique côté aspiration de la pompe tubulaire.

**8.** Procédé de fabrication d'une préparation médicamenteuse selon l'une des revendications précédentes, **caractérisé en ce qu'**à chaque étape de dosage, le récipient cible (3) est pesé et la quantité de liquide respectivement transférée est ainsi vérifiée.

**9.** Procédé de fabrication d'une préparation médicamenteuse selon l'une des revendications précédentes, **caractérisé en ce que** la quantité de liquide acheminée par la pompe tubulaire (6) est calculée et le récipient cible (3) est pesé, pour vérifier la quantité de liquide acheminée, la succession de différents liquides dans une arrivée du récipient cible (3) est prise en compte, pour calculer la masse spécifique du liquide lors de la vérification au moment du pesage.

**10.** Procédé de fabrication d'une préparation médicamenteuse selon l'une des revendications précédentes, **caractérisé en ce que** le récipient cible (3) est pesé à chaque étape de dosage individuelle, vérifiant ainsi la quantité de liquide transférée dans le récipient cible (3) à chaque étape de dosage individuelle.

**11.** Procédé de fabrication d'une préparation médicamenteuse selon l'une des revendications précédentes, **caractérisé en ce que** la quantité de liquide transférée dans le récipient cible (3) lors d'une étape de dosage est calculée en tenant compte de la courbe caractéristique côté pression de la pompe tubulaire (6).

**12.** Procédé de fabrication d'une préparation médicamenteuse selon l'une des revendications précédentes, **caractérisé en ce que** le débit de la pompe tubulaire (6) est vérifié à l'aide d'un capteur de débit (16) et/ou **en ce qu'**un capteur de bulles (17) est utilisé pour vérifier qu'il n'y a pas de bulles dans une arrivée vers le récipient cible (3).

**13.** Procédé de fabrication d'une préparation médicamenteuse selon l'une des revendications précédentes, **caractérisé en ce qu'**un facteur de dosage de la pompe tubulaire (6) est déterminé dans une étape d'étalonnage préalable par pesage d'un récipient cible (3) et/ou **en ce que**, dans une étape de dosage à partir d'un récipient source, dans lequel une roue de pompe (8) de la pompe tubulaire (6) tourne au moins un tour complet, le facteur de dosage de la pompe est étalonné.

**14.** Procédé de fabrication d'une préparation médicamenteuse selon l'une des revendications précédentes, **caractérisé en ce qu'**un kit de transfert est utilisé pour transférer les liquides des récipients sources (2) dans le récipient cible (3), lequel comprend une unité de soupape (5), un tuyau (14), qui peut être inséré dans la pompe tubulaire, et comprend une pluralité de tuyaux (15) pour le raccordement des récipients sources (2).

**15.** Installation pour la fabrication d'une préparation médicamenteuse, notamment installation pour la fabrication d'aliments parentéraux, comprenant une pompe tubulaire et **caractérisée par** un système avec une commande électronique pour mettre en oeuvre un procédé selon l'une des revendications précédentes 1 à 14 ci-dessus.

Fig. 1

Fig. 2

Fig. 3

Fig. 4a

22

22

15

15

Fig. 4b

23

14

Fig. 4c

1. Quellbehälter anschließen und Wastebag als Zielbehälter einlegen

2. Entlüften aller Zu- und Ablaufschläuche durch Transferieren der Flüssigkeiten aus den Quellbehältern in den Wastebag

3. Bestimmen des Dosierfaktors der Schlauchpumpe durch Wiegen des Wastebag beim Pumpen von UI

4. Wastebag verwerfen und Zielbehälter anschließen

5. Verbringen des Pumpenrades in einen Bereich mit saugseitig linearer Kennlinie durch Pumpen von UI

6. Dosierung einer Mikromenge

7. Wiegen des Zielbehälters zur Überprüfung der dosierten Mikromenge unter Berücksichtigung des spezifischen Gewichts der in den Zielbehälter transferierten Flüssigkeit

Fig. 5a

8. Dosierung eines Hauptbestandteils unter Berücksichtigung der saugseitigen Kennlinie der Schlauchpumpe

9. Wiegen des Zielbehälters zur Überprüfung der dosierten Menge unter Berücksichtigung des spezifischen Gewichts der in den Zielbehälter transferierten Flüssigkeit

10. Dosierung weiterer Mikromengen und/oder Hauptbestandteile

11. Spülen des Transfer-Set mit UI und Kalibrieren des Dosierfaktors der Schlauchpumpe durch Wiegen des Zielbehälters

12. Entnahme des Zielbehälters nach Abschluss aller Dosierschritte

Fig. 5b

Fig. 6

Fig. 7

Wiegen des Zielbehälter bei einem
Dosierschritt

Erster
Grenzbereich
eingehalten

nein

Abbruch des
Befüllvorgangs
und
Fehlermeldung

Ja

Zweiter engerer
Grenzbereich
eingehalten

nein

ja

Fortsetzen des
Befüllvorgangs,
dann Meldung
dass Anlage
entlüftet
werden muss

Fortsetzen des
Befüllvorgangs,
dann kann
nächster
Zielbehälter
eingelegt
werden

Fig. 8

Berechnung des in den Zielbehälter transferierten
Volumens auf Basis der druckseitigen
Pumpenkennlinie

Bestimmung der in den Zielbehälter transferierten
Flüssigkeit oder Flüssigkeiten und deren Dichte

Berechnung des Gewichtes der beim Dosierschritt
in den Zielbehälter transferierten Flüssigkeit

Fig. 9

Kontinuierliches Überwachen des Flusses mittels des Blasensensors

Erster Grenzbereich eingehalten

nein

Abbruch des Befüllvorgangs und Fehlermeldung

Ja

Zweiter engerer Grenzbereich eingehalten

nein

Fortsetzen des Befüllvorgangs, dann Meldung dass Anlage kalibriert werden muss

ja

Fortsetzen des Befüllvorgangs, nach Abschluss kann nächster Zielbehälter eingelegt werden

Fig. 10

Kontinuierliches Überwachen der Fließgeschwindigkeit

Berechnen der von der Schlauchpumpe erzeugten Fließgeschwindigkeit

Vergleich der Fließgeschwindigkeiten

Abweichung von mehr als 20 %

ja

nein

Abbruch des Befüllvorgangs und Fehlermeldung

Fortsetzen des Befüllvorgangs

Fig. 11

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5697407 A